# EUROPEAN PATENT SPECIFICATION

(11) **EP 1 276 501 B1**
(45) Date of publication and mention of the grant of the patent: **21.12.2005**
(21) Application number: 01928938.8
(22) Date of filing: 25.04.2001
(51) Int. Cl.: A61K 41/00, A61P 35/00

(54) **METHOD FOR TREATMENT OF TUMORS USING PHOTODYNAMIC THERAPY**
BEHANDLUNG VON TUMOREN MIT PHOTODYNAMISCHER THERAPIE
PROCEDE DE TRAITEMENT DES TUMEURS PAR THERAPIE PHOTODYNAMIQUE

(30) Priority: 25.04.2000 US 199545 P
(43) Date of publication of application: 22.01.2003
(73) Proprietor: IMMUNEX CORPORATION, Thousand Oaks, CA 91320-1799 (US)
(72) Inventor: FANSLOW, William, C., III, Normandy Park, WA 98166 (US); THOMAS, Elaine, K., Seattle, WA 98112 (US)
(74) Representative: Bassil, Nicholas Charles
(86) International application number: PCT/US2001/013616
(87) International publication number: WO 2001/080888

(56) References cited:
- WO-A-00/51638
- WO-A-97/12633
- WO-A-98/01538
- US-A- 5 962 406
- DATABASE CA [Online] CHEMICAL ABSTRACTS SERVICE, COLUMBUS, OHIO, US; KING, D. E. ET AL: "Photodynamic alteration of the surface receptor expression pattern of murine splenic dendritic cells" retrieved from STN Database accession no. 131:2249 XP002194261 & SCAND. J. IMMUNOL. (1999), 49(2), 184-192 ,

### SEQUENCE LISTING

<110> Immunex Corporation
   Panslow III, William C.
   Thomas, Elaine K.
<120> METHOD FOR TREATMENT OF TUMORS USING PHOTODYNAMIC THERAPY
<130> 2922-WO
<140> --to be assigned--
   <141> 2001-04-24
<160> 3
<170> PatentIn Ver. 2.0
<210> 1
   <211> 260
   <212> PRT
   <213> Mus sp.
<400> 1
   Met Ile Glu Thr Tyr Ser Gln Pro Ser Pro Arg Ser Val Ala Thr Gly
   1 5 10 15
   Leu Pro Ala Ser Met Lys Ile Phe Met Tyr Leu Leu Thr Val Phe Leu
   20 25 30
   Ile Thr Gln Met Ile Gly Ser Val Leu Phe Ala Val Tyr Leu His Arg
   35 40 45
   Arg Leu Asp Lys Val Glu Glu Glu Val Asn Leu His Glu Asp Phe Val
   50 55 60
   Phe Ile Lys Lys Leu Lys Arg Cys Asn Lys Gly Glu Gly Ser Leu Ser
   65 70 75 80
   Leu Leu Asn Cys Glu Glu Met Arg Arg Gln Phe Glu Asp Leu Val Lys
   85 90 95
   Asp Ile Thr Leu Asn Lys Glu Glu Lys Lys Glu Asn Ser Phe Glu Met
   100 105 110
   Gln Arg Gly Asp Glu Asp Pro Gln Ile Ala Ala His Val Val Ser Glu
   115 120 125
   Ala Asn Ser Asn Ala Ala Ser Val Leu Gln Trp Ala Lys Lys Gly Tyr
   130 135 140
   Tyr Thr Met Lys Ser Asn Leu Val Met Leu Glu Asn Gly Lys Gln Leu
   145 150 155 160
   Thr Val Lys Arg Glu Gly Leu Tyr Tyr Val Tyr Thr Gln Val Thr Phe
   165 170 175
   Cys Ser Asn Arg Glu Pro Ser Ser Gln Arg Pro Phe Ile Val Gly Leu
   180 185 190
   Trp Leu Lys Pro Ser Ser Gly Ser Glu Arg Ile Leu Leu Lys Ala Ala
   195 200 205
   Asn Thr His Ser Ser Ser Gln Leu Cys Glu Gln Gln Ser Val His Leu
   210 215 220
   Gly Gly Val Phe Glu Leu Gln Ala Gly Ala Ser Val Phe Val Asn Val
   225 230 235 240
   Thr Glu Ala Ser Gln Val Ile His Arg Val Gly Phe Ser Ser Phe Gly
   245 250 255
   Leu Leu Lys Leu
   260
<210> 2
   <211> 261
   <212> PRT
   <213> Homo sapiens
<400> 2
<210> 3
   <211> 33
   <212> PRT
   <213> PEPTIDE
<400> 3
   Arg Met Lys Gln Ile Glu Asp Lys Ile Glu Glu Ile Leu Ser Lys Ile
   1 5 10 15
   Tyr His Ile Glu Asn Glu Ile Ala Arg Ile Lys Lys Leu Ile Gly Glu
   20 25 30
   Arg

## Description

### FIELD OF THE INVENTION

The present invention describes methods for treating tumors. In particular, the present invention involves treating tumor-bearing individuals with photodynamic therapy in combination with additional reagents.

### BACKGROUND OF THE INVENTION

Photodynamic therapy (PDT) is a treatment for cancer that involves the use of a photosensitizer and light. In this treatment modality, an individual afflicted with cancer or precancerous condition is administered a photosensitizing agent. Cancerous (and precancerous) cells retain the photosensitizer more readily than normal tissues. Subsequent exposure of the cells to wavelength-specific light induces a photochemical reaction that causes oxidative damage to numerous cellular components and cell death (reviewed by Dougherty et al., *J. Natl. Cancer Institute* 90:889; 1998).

CD40 is a transmembrane protein expressed on various normal cells, including B lymphocytes, monocytes some epithelial cells and dendritic cells, as well as on various transformed carcinoma cell lines (Clark, *Tissue Antigens,* 36:33 (1990). A ligand for CD40 is expressed on activated T cells (Spriggs et al, *J*. *Exp. Med.,* 176:1453 (1992); Armitage et al, *Nature,* 357:80 (1992). Binding of CD40 with CD40L causes B cell proliferation in the absence of any co-stimulus, and induction of antibody secretion from B cells in the presence of cytokines.

Soluble forms of CD40L and agonistic CD40 antibodies (i.e., those that mimic the biological effects of CD40L) are useful in the treatment of diseases characterized by neoplastic cells that express CD40, such as B lymphomas, melanomas and carcinomas (U.S. Patent 5,674,492). Soluble CD40L has also been used to promote the proliferation and/or differentiation of CD40-positive sarcoma cells, as a means of directly treating the malignancy or as an adjunct to chemotherapy, or to increase the immune response of an immunosuppressed individual, such as a subject suffering from malignancy (U.S. Patent 5,945,513). Moreover, soluble CD40L has been used to stimulate a T effector cell-mediated immune response (WO96/26735).

### SUMMARY OF THE INVENTION

An object of the present invention is to provide a kit of parts comprising a CD40 binding protein and a photosensitizer for separate, simultaneous or subsequent administration to a subject for the treatment of a tumour or a precancerous-condition, as defined by the appended claims.

Another object of the present invention is the use of a CD40 binding protein in the preparation of a medicament for the photodynamic treatment of a subject suffering from a tumour or pre-cancerous condition, as defined by the appended claims.

These and other objects of the present invention, which will be apparent for the detailed description of the present invention provided hereinafter, have been met, as defined by the appended claims.

The present invention further describes the above identified methods for treating tumor-bearing subjects and methods for inducing a CTL response that further include administering additional therapeutic or active agents. Such therapeutics or active agents include those that induce tumor cell death and/or apoptosis, those that increase the numbers of antigen-presenting cells, those that stimulate maturation of dendritic cells and those that lead to T effector cell expansion and immune activation. Suitable additional therapeutic or active agents include FasL, TRAIL, TNF alpha and CD30L.

The present invention further describes, in combination with the above described Kit of parts or use, *in vivo* and/or *in vitro* methodologies that involve immune based tumor therapy and/or dendritic cell expansion and maturation techniques for optimizing anti-tumor therapeutic effects of PDT and CD40L. More particularly, the present invention describes methods for treating tumor-bearing individuals that involve administering Flt3L to the tumor bearing individual; administering photodynamic therapy to the individual, and administering CD40L to the individual. Additional therapeutic or active agents that induce tumor cell death and/or apoptosis, increase the numbers of antigen-presenting cells and stimulate dendritic cell maturation can be administered as well. The present invention further describes *in vitro* methodologies that involve collecting dendritic cells from the individual, expanding the dendritic cells by exposing them to Flt3-L, infusing the expanded dendritic cells into the individual, treating the individual with photodynamic therapy and administering CD40 binding protein to the individual. Prior to collecting the dendritic cells, administering flt3-L to the individual will aid in dendritic cell mobilization and increase the number of dendritic cells available for collection. Alternatively, *in vitro* methods can include collecting hematopoietic stem or progenitor cells and contacting the cells with flt3-L to generate dendritic cells, prior to infusing the generated dendritic cells into the tumor bearing individual.

A variety of CD40 binding proteins may be employed in the present invention, including, for example, an antibody that binds CD40; full-length-membrane bound CD40L; a soluble extracellular region of a CD40L; a fusion protein comprising a CD40 binding region (or domain) from a CD40L or an antibody to CD40, fused to a second protein, for example, an immunoglobulin Fc domain or a zipper domain.

Suitable CD40 antibodies include CD40 antibodies that bind and crosslink CD40, thereby transducing a signal. Among these are monoclonal antibody HuCD40-M2 (ATCC HB 11459) and CD40 binding proteins comprising an antigen-binding domain derived from antibody HuCD40M2.

### DETAILED DESCRIPTION OF THE INVENTION

It is believed that in the present invention, a tumor killing or lysing procedure known as Photodynamic therapy (PDT) induces cell death, and leads to antigen uptake and presentation by dendritic cells (DC) in sites draining the dying tumor. When contacted with a CD40 binding protein, these tumor antigen-bearing DC induce a potent memory CTL response specific to the tumor. The CTL response leads to eradication or significant reduction of the remaining tumor burden. The methods described herein can be used to treat a wide range of tumors and precancerous cells, including, but not limited to, basal and squamous cells, skin cancers, breast cancer, cancers that are metastatic to skin, brain tumors, head and neck, stomach, and female genital tract malignancy, cancers and precancerous conditions of the esophagus such as Barrstt's esophagus.

The present invention describes combining PDT with administering CD40 binding protein in a tumor bearing subject. In another embodiment, the methods described by the present invention further include combination therapies of administering one or more active agents for enhancing immune-based tumor therapy. More particularly, in addition to administering CD40 binding protein in combination with PDT, the present invention describes administering one or more mobilization agents for increasing dendritic cells numbers; and/or administering one or more agents for inducing dendritic cell maturation; and/or administering one or more agents which stimulate T cell proliferation.

Dendritic cells can be increased *in vivo* by administering Flt3L and/or GM-CSF to the tumor bearing subject. Suitable agents for inducing dendritic cell maturation include CD40L, TNF alpha, RANKL, LPS, and conditioned monocyte media. Dendritic cell maturation agents can be administered systemically or locally, at or near the tumor site. Suitable agents for stimulating T cell proliferation and function include, but are not limited to, IL-2, IL-15, IL-7, lL-12, and IFN gamma. Agents that stimulate T cell proliferation may be administered systemically or in the vicinity of the tumor or the draining lymph nodes.

In addition to, or as an alternate to the *in vivo* methods for generating dendritic cells, dendritic cells can be generated using *in vitro* methods to be subsequently administered to the tumor-bearing subject. For example, CD34+ cells can be collected, utilizing known collection and cell separation methods, subsequent to *in vivo* mobilization with Flt3L, G-CSF, GM-CSF, SCF or cyclophosphamide, and/or other mobilization agents. Dendritic cells from the collected CD34+ cells can be grown *in vitro* using dendritic cell generation active agents such as Flt3L, GM-CSF, CD40L, and IL-15. Alternatively, PBMC can be collected for the purpose of generating dendritic cells *in vitro,* optionally using reagents such as GM-CSF and IL-4 to generate the dendritic cells.. The *in vitro* generated dendritic cells may be infused into the PDT receiving tumor-bearing subject in order to increase the number of dendritic cells for inducing a CTL response.

Methods for the *in vivo* and *in vitro* mobilization and generation of dendritic cells and methods for stimulating T cell proliferations are described in WO 97/12633 and copending U.S. applications S/N 09/154,903, 09/444,027, 09/448,378.

### Photodynamic Therapy

The tumor killing or lysing procedure utilized in the present invention, PDT, is a cancer treatment that utilizes a photochemical reaction to destroy neoplastic cells and cells that are pre-cancerous or precursors to neoplastic cells (reviewed by Dougherty et al., *J. Natl. Cancer Institute* 90:889; 1998. The application of PDT is according to methods known in the art which generally involve administering one or more photosensitizers to a tumor bearing subject followed by a light activation step in which light of a specific wavelength is directed to the tumor where the photosensitizer is lodged.

The cytotoxic effect of PDT is primarily mediated by the formation of singlet oxygen generated by energy transfer from a light-activated, tissue localized photosensitizer to ground state oxygen. Singlet oxygen has a short radius of action, and can cause oxidative damage to numerous cellular components at or near the site of its generation (Gollnick et al, *Cancer Res.,* 57:3904-3909 (1997)).

The oxidative damage mediated by PDT has a variety of effects on tumor cells, the microvasculature within and near the tumor, and on cells of the immune system. PDT induces changes in the plasma membrane and membranes of cellular organelles of affected cells, upregulating expression of some stress protein genes and activating certain genes involved in apoptosis, as well as leading to the release of powerful inflammatory mediators. Although the role of the various effects induced by PDT is not clear, it is believed that the combination of the effects is necessary for eradication of cancer cells (Dougherty et al., *supra).*

The acute phase immune reaction to PDT is inflammatory in nature; control of tumors over the long term, however, appears to be a result of specific anti-tumor immunity. The effectiveness of such long-term, tumor-specific immunity is unpredictable because PDT can significantly suppress certain immune functions, especially those involving effector T cells (Elmets et al, *Cancer Res.,* 46:1608-1611 (1986); Simkin et al, *Proc. Int. Soc. Optical Eng.,* 2392:2333 (1995); Gruner et al, *Scand. J. Immunol.,* 21:267-273 (1985)). The suppressive effects are believed to involve mediation of immune-modulating cytokines, such as IL-6 and IL-10 (Gollnick et al, *supra).*

PDT has been used effectively in the treatment of a variety of human tumors and precancerous conditions, including basal and squamous cells, skin cancers, breast cancer, metastatic to skin, brain tumors, head and neck, stomach, and female genital tract malignancy, cancers and precancerous conditions of the esophagus such as Barrett's esophagus (U.S. Patent 6,013,053, Marcus, In: *Future Directions and Applications in Photodynamic Therapy,* Gomer, Ed., Bellingham, WA SPIE Optical Engineering Press (1990) pages 5-56; and Overholt et al, *Sem. Surg. Oncol.,* 11:1-5 (1995)).

Examples of useful photosensitizer which can be employed in the present invention include hematoporphyrins (Kessel, *Cancer Lett.,* 39:193-198 (1988), uroporphyrins, phthalocyanines (Kreimer-Birnbaum, *Sem. Hematol.,* 26:157-173 (1989), purpurins (Morgan et al, *Photochem. Photobiol.,* 51:589-592 (1990); and Kessel, *Photochem. Photobiol.* 50:169-174 (1989), acridine dyes, bacteriochlorophylls (Beems et al, *Photochem. Photobiol.,* 46:639-643 (1987); and Kessel et al, *Photochem. Photobiol.,* 49:157-160 (1989), and bacteriochlorins (Gurinovich et al, *J*. *Photochem. Photobiol. B-Biol.,* 13:51-57 (1992)).

Photosensitizers suitable for use in the present invention include those summarized, in part, in Table 1 of U.S. Patent 5,942,534. An alternative to administration of the photosensitizer itself, is administration of a precursor of that compound. For example, 5-aminolevulinic acid causes endogenous production of the photosensitizer protoporphyrin IX (Morgan et al, *J*. *Med. Chem. ,* 32:904-908 (1989).

### CD40/CD40 Binding Proteins

CD40 is a member of the tumor necrosis factor (TNF)/nerve growth factor (NGF) receptor family that has been found to be expressed on B lymphocytes, monocytes, some epithelial cells and dendritic cells (Clark, *Tissue Antigens,* 36:33; 1990). This cell surface antigen has been shown to play an important role in B cell proliferation and differentiation, and in the growth of malignant cells upon which it is expressed.

The ligand for CD40 (hereinafter "CD40L") has been identified and characterized, and DNA encoding the same has been cloned from peripheral blood T cells (Spriggs et al, *J. Exp. Med,* 176:1453 (1992); Armitage et al, *Nature,* 357:80 (1992); and Armitage et al, U.S. Patent Nos. 5,961,974, 5,962,406 and 5,981,724. CD40L biological activity is mediated by binding of this cytokine with CD40, and includes B cell proliferation in the absence of any co-stimulus, and induction of antibody secretion from B cells, in the presence of cytokines.

As used herein, "CD40 binding protein" refers to polypeptides that specifically bind CD40 in a noncovalent interaction based upon the proper conformation of the CD40 binding protein and CD40 itself. Preferably, the CD40 binding protein has agonistic activity, that is, it mimics the native ligand for CD40 (CD40L) that is present on activated T cells by binding to, and transducing a signal to, a cell expressing CD40. Assays for biological activities of CD40L are useful for assessing agonistic activity. Additional methods to measure agonistic activity of a CD40 binding protein include analyzing CD40 binding protein for the ability to inhibit binding of CD40 to CD40L. CD40 binding proteins that bind CD40 and inhibit binding of CD40 to CD40L, as determined by observing at least about 90% inhibition of the binding of soluble CD40 to CD40L, will have agonistic activity.

The CD40 binding proteins useful in the present invention include antibodies to CD40 (including humanized antibodies or antibodies that have been manipulated through recombinant means to render them suitable for therapeutic use), CD40L, soluble CD40L, and fusion proteins comprising a soluble CD40L or an antibody to CD40, and a second protein. More particularly, CD40 binding proteins include antibodies to CD40 that crosslink CD40 and transduce a signal; full-length CD40L; oligomeric soluble forms of CD40L or fragments thereof that bind CD40 (e.g. the CD40L extracellular domain and fragments thereof); CD40L fusion proteins, e.g. soluble CD40L/Fc fusions and soluble CD40L/leucine zipper fusions. Oligomeric soluble forms of CD40L include the extracellular domain of CD40L or fragments of the extracellular domain that bind CD40 that are in oligomeric form. One such example of soluble oligomeric CD40L is the extracellular domain fragment of amino acids 113-261 of SEQ ID NO:2 and the leucine zipper of SEQ ID NO:3. When the fragment and the leucine zipper are combined, an oligomeric form of CD40L results.

Full length CD40L includes polypeptides comprising amino acids 1 through 260 of SEQ ID NO:1 and amino acids 1 through 261 of SEQ ID NO:2. Soluble forms of CD40L include amino acids 47 through 260, 113 through 260, and 120 through 260 of SEQ ID NO:1 and amino acids 47 through 261, 112 through 261, 113 through 261, and 120 through 261 of SEQ ID NO:2. Further, CD40 binding proteins include fragments of the extracellular domain of CD40L (SEQ ID NO:1 and SEQ ID NO:2) that bind CD40. Such binding is sufficient to inhibit binding of soluble CD40 to CD40L, as determined by observing at least about 90% inhibition of the binding of soluble CD40 to CD40L.

Alternative embodiments of CD40L polypeptide, soluble CD40L polypeptides and suitable fragments thereof include polypeptides in which a cysteine at amino acid 194 of SEQ ID NO:2 is substituted with tryptophan. Still additional embodiments are encompassed by CD40L polypeptide and soluble CD40L polypeptides that are encoded by the complement of DNA that hybridizes to a DNA encoding any of the aforementioned polypeptides under conditions of severe stringency (hybridization in 6 X SSC at 63°C overnight; washing in 3 X SSC at 55°C) and which binds soluble CD40. Such binding is sufficient to inhibit binding of soluble CD40 to CD40L, as determined by observing at least about 90% inhibition of the binding of soluble CD40 to CD40L.

A preferred CD40 binding protein is an oligomeric soluble CD40L in which the soluble portion is an oligomerized extracellular domain fragment of SEQ m NO:2 and the cysteine at amino acid 194 is substituted with tryptophan. Preferably, the oligomeric soluble CD40L includes an oligomerizing zipper domain (e.g. leucine zipper) such as that of SEQ ID NO:3 or a variant peptide in which conservative amino acid substitutions have been made, wherein the peptide is capable of forming an oligomeric soluble CD40L fusion protein. One such soluble oligomeric CD40L/leucine zipper fusion protein includes a polypeptide having amino acids 113-261 of SEQ ID NO:2 and the leucine zipper of SEQ ID NO:3 (CD40L/LZ).

Methods for expression of recombinant CD40L polypeptides are also described in the Armitage patents. Similar methods may be used for expression of other CD40 binding proteins. Moreover, numerous expression systems are known to those of routine skill in the art of molecular biology, including prokaryotic and eukaryotic expression systems. The expression system selected may affect the nature of the recombinant CD40 binding protein expressed. For example, CD40L expressed in mammalian expression systems (e.g., COS7 cells) may be similar to a native CD40L in molecular weight and glycosylation pattern, whereas CD40L expressed in yeast may be more highly glycosylated than native CD40L. Expression of CD40L in bacterial expression systems, such as *E*. *coli,* provides non-glycosylated molecules.

Antibodies to CD40 which can be employed in the present invention may be polyclonal or monoclonal. The particular agonistic CD40 antibody employed in the present invention is not critical thereto. Examples of such CD40 antibodies include HuCD40-M2 (ATCC No. HB11459) and HuCD40-M3, and antigen binding domains thereof. Additional CD40 mAbs which can be employed in the present invention may be generated using conventional techniques (see U.S. Patents RE 32,011, 4,902,614, 4,543,439, and 4411,993. Useful agonistic antibodies may also be constructed utilizing recombinant DNA techniques to "humanize" a murine antibody, or prepare single-chain antibodies, as described in U.S. Patent 5,801,227.

Once suitable CD40 binding proteins have been obtained, they may be isolated or purified by many techniques well known to those of ordinary skill in the art. Suitable techniques include peptide or protein affinity columns, HPLC or RP-HPLC, purification on protein A or protein G columns, or any combination of these techniques. Recombinant CD40 binding proteins can be prepared according to standard methods, and tested for binding specificity to the CD40 utilizing assays known in the art, including for example ELISA, ABC, or dot blot assays, as well by bioactivity assays such as those described for CD40 mAb.

### Administration of CD40 Binding Protein

The CD40 binding protein may be administered in a suitable diluent or carrier to a subject, preferably a human. Thus, for example, CD40 binding protein can be given by bolus injection, subcutaneous or IP, continuous infusion, intermittent IV infusion, sustained release from implants, or other suitable technique.

Typically, a CD40 binding protein will be administered in the form of a pharmaceutical composition comprising purified CD40 binding protein in conjunction with physiologically acceptable carriers, excipients or diluents. Such carriers are nontoxic to subjects at the dosages and concentrations employed. Ordinarily, the preparation of such compositions entails combining a CD40 binding protein with buffers, antioxidants such as ascorbic acid, low molecular weight (less than about 10 residues) polypeptides, proteins, amino acids, carbohydrates including glucose, sucrose or dextrans, chelating agents such as EDTA, glutathione and other stabilizers and excipients. Neutral buffered saline or saline mixed with conspecific serum albumin are exemplary appropriate diluents.

The particular therapeutically effective amount employed is not critical to the present invention, and will vary depending upon the particular CD40 binding protein selected, the type, frequency and intensity of PDT, as well as the age, weight and sex of the subject. Typically, therapeutically effective dosages, (doses that provide anti-neoplastic activity or doses sufficient to provide an enhanced CTL response) of CD40 binding proteins will be in the range of from about 0.01 to about 1.0 mg/kg body weight. More typically doses are in the range of 0.05 to 0.2 mg/kg bodyweight. As described below, administering CD40 binding protein can be carried out one or more days prior to administering PDT, continuing for a period of time in which the enhanced CTL response and enhanced immune response and/or enhanced antigen presenting cell maturation is effective. Alternatively administering CD40 binding protein can commence the day of or days following PDT. In any case, it is preferred that CD40 binding protein be present to enhance an immune response concurrent with or immediately following PDT.

CD40 binding proteins may also be used in conjugates of, or combination with, drugs, toxins or radioactive compounds. Preparation of such conjugates for treatment of various diseases are known in the art (see, for example, Waldmann, *Science,* 252:1657 (1991)).

### Administration of PDT

Photodynamic therapy, or PDT, is carried out by methods known in the art. Methods for administering PDT are described in Dougherty et al., *J. Natl. Cancer Institute* 90:889; 1998. Such methods include administering a photosensitizer or a mixture of photosensitizers, followed by exposure of the subject (the affected body area) to light that is absorbed by the photosensitizer. Subsequent to absorbing the light, the photosensitizer becomes excited and causes the generation of singlet oxygen. Singlet oxygen is highly toxic, but has a short radius of action. Various modes of administering a photosensitizer are known in the art, and will be useful in the present invention. For example, the photosensitizer may be administered orally, topically, parenterally, or locally (i.e., directly into or near the tumor or precancerous area). The photosensitizers may also be delivered using vehicles such as phospholipid vesicles or oil emulsions. Use of lipid-based delivery vehicles may result in enhanced accumulation of the photosensitizer in neoplastic cells. Alternative methods of delivery also encompassed in the instant invention include the use of microspheres, or monoclonal antibodies or other proteins that specifically bind a protein (or proteins) located on the surface of neoplastic cells.

The particular photosensitizer employed is not crucial to the present invention. Examples of photosensitizers useful in the present invention include hematoporphyrins , uroporphyrins, phthalocyanines, purpurins, acridine dyes, bacteriochlorophylls, bacteriochlorins and others are disclose herein. A preferred photosensitizer employed is Photofrin® (QLT, Vancouver, Canada); additional examples are disclosed herein, and discussed in Dougherty et al. as well as various other resources disclosed herein.

The amount of photosensitizer administered will vary depending upon the particular photosensitizer employed, the age, weight and sex of the subject, the mode of administration, as well as the type, size and location of the tumor. For example, Photofrin® can be used at doses of 2.0 or 2.5 mg per kg body weight. The dosing for other types of photosensitizers can vary, ranging from 0.3 to 7.2 mg per kg body weight. Accordingly, those of skill in the art are able to determine preferred doses of various photosensitizing agents after examination of the relevant dosing information from the manufacturer and/or other experts in the field.

The wavelength of light to which the subject is exposed will vary depending upon the photosensitizer employed, and the location and depth of the tumor or precancerous cells. Generally, the subject will be exposed to light having a wavelength of about 600 to 900 nm, preferably about 600 to about 640 nm for Photofrin®. Several other photosensitizing agents have stronger absorbances at higher wavelengths, from about 650 to 850 nm, which can be beneficial for deeper tumors because light of longer wavelength tends to penetrate further into tissue. Conversely, a wavelength of about 410 nm may give better results when shallow penetration is desired; such dosages also fall within the scope of this invention.

The dose of light to which the subject is exposed will vary depending upon the photosensitizer employed. Generally, the subject will be exposed to light dose of about 50 to 500 J/cm2 of red light, for Photofrin®. Other sensitizers may be more efficient, and thereby require smaller fluences, typically about 10 J/cm2. At higher fluences, hyperthermia may occur, which can enhance PDT; moreover, hyperthermia and PDT may act synergistically. Several different light sources are known in the art; any suitable light source capable of delivering an appropriate dosage of a selected wavelength may be used in the inventive methods.

The timing of light exposure will depend on the photosensitizer used, the nature and location of the tumor or precancerous cells, and the methods of administration. Typically, light exposure occurs at about one hour to four days after administration of the photosensitizer. Moreover, shorter time periods may be used, again depending on the photosensitizer, and the nature and location of the tumor. For example, light exposure after topical administration of a photosensitizer may occur as early as about ten minutes, or at about three hours after administration (see U.S. Patent 6,011,563).

### Enhancing Immune-based Tumor Therapy with Combination Therapies

The Kit of parts and use of the present invention further include one or more active agents to be administered for enhancing immune-based tumor therapy. More particularly, in addition to administering CD40 binding protein in combination with PDT, the present invention describes administering one or more mobilization agents for increasing dendritic cell numbers; and/or administering one or more agents for inducing dendritic cell maturation; and/or administering one or more agents which stimulate T cell proliferation, T effector cell expansion and immune activation.

Dendritic cells can be increased *in vivo* by administering Flt3L (described in U.S. Patent No. 5,554,512) and/or GM-CSF to the tumor-bearing subject. For example, prior to administering PDT to a tumor bearing individual, Flt3-L can be administered for a period of between about 2 days to 18 days and preferable for from 10 to 14 days at a dose of 5 µg/kg to 250 µg/kg and preferably from 25 µg/kg to 150 µg/kg per day. Alternatively, Flt3L can be administered at levels ranging from 50 µkg to 450 µ/kg every 5 days.

In addition to, or as an alternate to *in vivo* methods for generating dendritic cells, dendritic cells can be generated using *in vitro* methods and subsequently administered to the tumor-bearing subject. For example, prior to administering PDT and subsequent to using *in vivo* mobilization with Flt3L, G-CSF, GM-CSF, cyclophosphamide, SCF and/or other mobilization agents, CD34+ cells, stem or progenitor cells can be collected utilizing known collection and cell separation. Dendritic cells from the collected CD34+, stem or progenitor cells can be grown *in vitro* using dendritic cell generation active agents such as Flt3L, GM-CSF, CD40L or other CD40 binding protein, and IL-15. Alternatively, PBMC can be collected for the purpose of generating dendritic cells *in vitro.* The *in vitro* generated dendritic cells may be infused into the PDT receiving tumor-bearing subject in order to increase the number of dendritic cells for inducing a CTL response. Cell culture media that incorporate Flt3-L and/or other agents for the *in vitro* generation and mobilization of dendritic cells include these agents in quantities sufficient to maximize the number of dendritic cells for the later infusion into the tumor-bearing subject or precancerous bearing subject. Such amounts may range from 0.1 µg/mL to 5 µg/mL and typically are about 2 µg/mL.

Methods for the *in vivo* and *in vitro* mobilization and generation of dendritic cells and methods for stimulating T cell proliferations are described in WO 97/12633 and copending U.S. applications S/N 09/154,903, 09/444,027, 09/448,378.

In accordance with the present invention CD40 binding proteins may be administered to stimulate maturation of DC, enhancing their capabilities to stimulate an effective, specific, anti-tumor cytotoxic response. CD40 binding proteins may be used in conjunction with other DC-maturation factors, such as TNF-alpha, a ligand for the receptor activator of NF-kappaB (RANKL), and substances such as lipopolysaccharide. Moreover, agents that enhance a CTL response may be use in conjunction with a CD40 binding protein. Such agents include Interleukins 2, 15, 7 and 12, and interferons-gamma and - alpha. Dendritic cell maturation agents can be administered systemically or locally, at or near the tumor site. Doses of CD40 binding proteins and specifically oligomeric soluble forms of CD40L, can range from 0.01 mg/kg to 1 mg/kg, and are preferably in the range of 0.05 mg/kg to 0.2 mg/kg. Dosing frequency can range from every day, to every other day and may be limited to once per week when the mode of administration favors such frequency (e.g. by i.v. administration).

Use of a CD40 binding protein in conjunction with PDT, in accordance with the present invention, means that the CD40 binding protein may be administered before, during or after PDT. Preferably, a CD40 binding protein is to be administered after PDT, most preferably CD40 binding protein administration begins on the day of, or about one to two days after PDT administration. Furthermore, the combination of a CD40 binding protein and PDT may be supplemented by the use of additional active agents as described herein. Additional active agents may be administered at the same time as, before, or after, administration of CD40 binding proteins, as appropriate for the agent and desired result. For example, FasL, TRAIL, CD30L and TNF alpha may be administered concurrent with administering CD40 binding protein. The presence of these active agents in combination therapies enhances the tumor eradicating characteristics of the combination of CD40 binding protein and PDT. In one embodiment the active agent or active agents are to be administered intra-tumor or close to the tumor.

Because PDT is an entirely different process from radiotherapy (ionizing radiation), chemotherapy and surgery, and thus the use of PDT is not precluded by prior radiotherapy, chemotherapy or surgery (Hsi et al, *Drugs,* 57:7250734 (1999); and McCaughan, *Drugs & Aging,* 15:49068 (1999)), it can be used in conjunction with such processes (i.e., before, during or after an alternative process such as radiation therapy). The relatively low toxicity of PDT also makes it suitable as a repeatable form of therapy. Furthermore, the improvements described herein may render it possible to further reduce side effects, by decreasing the amount of photosensitizer or the dosage of light needed.

### Prevention or Treatment of Disease

These results presented herein indicate that CD40 binding proteins may be of significant clinical use in the treatment of various tumors. The term treatment, as it is generally understood in the art, refers to initiation of therapy after clinical symptoms or signs of disease have been observed. In one embodiment, the tumor may express CD40, for example, B lymphomas, melanomas or sarcomas. In another embodiment, the tumor does not express CD40. Examples of such tumors include T cell lymphomas and leukemias, many connective tissue tumors, and neuroblastomas.

Furthermore, the present invention will be useful in the treatment of precancerous conditions (such as Barrett's esophagus) for which PDT can be employed. When employed in this manner, the inventive methods described herein may be thought of as preventative measures rather than strictly defined treatment of an afflicted individual.

The present invention may be used in conjunction with other therapies appropriate for afflicted subjects, including chemotherapy, radiation therapy, and immunotherapy.

The following examples are intended to illustrate particular embodiments, and not limit the scope, of the invention.

### EXAMPLE 1

This example demonstrates that the protective antitumor response induced by PDT *in vivo* is dependent on the CD40:CD40L interaction. Female BALB/c mice (n=45) were inoculated subcutaneously (SQ) with 5.0 x 10⁴ BALB/c mouse mammary carcinoma EMT6 cells. On day 6, post tumor inoculation, 30 of the tumor-bearing mice were injected intraperitoneally (IP) with Photofrin® (a photosensitizer obtained from QLT, Vancouver, Canada) at a dose of 5.0 mg/kg. The remaining 15 tumor-bearing mice were not given Photofrin (negative control). The following day (day 7), 15 of the Photofrin-injected tumor-bearing mice were treated with 135 J/cm² of red light having a wavelength of 630 nm. Tumors from the remaining 15 Photofrin-injected tumor-bearing mice, which were not exposed to the red light, were surgically removed. Immediately following light treatment or surgery, 5 mice of each treatment group received IP, 200 µg of rat IgG (Sigma), 200 µg of rat monoclonal anti-muCD40L M158 (Immunex Corporation, Seattle, WA), or no antibody injection. These antibody injections were repeated on days 8, 10 and 12. The negative control mice group was similarly injected with antibodies on days 7, 8, 10 and 12. On day 13, lymph node cells from all of the treatment groups were isolated and mixed with fresh EMT6 tumors cells at a ratio of 500 lymph node cells to 1 tumor cell, i.e, 2.5 x 10⁶ lymph node cells and 5.0 x 10⁴ EMT6 cells, and the resulting mixture was injected SQ into non-tumor bearing BALB/c mice. Tumor incidence and tumor growth was monitored from day 18 to day 90. The results are shown in Table 1 below.

**Table 1:**

| Role of CD40/CD40L Interaction in the Protective Anti-tumor Response Induced by PDT | | |
|---|---|---|
| Tumor Therapy | Antibody Treatment | Tumor take (%incidence) at day 90 |
| None | None | 5/5 (100%) |
| None | Rat IgG | 4/5 (80%) |
| None | Anti-CD40L M158 | 5/5 (100%) |
| PDT | None | 1/5 (20%) |
| PDT | Rat IgG | 0/5 (0%) |
| PDT | Anti-CD40L M158 | 3/5 (60%) |
| Surgical removal | None | 4/5 (80%) |
| Surgical removal | Rat IgG | 5/5 (100%) |
| Surgical removal | Anti-CD40L M158 | 4/5 (80%) |

As shown in Table 1, treatment of tumor-bearing mice with PDT induced a strong anti-tumor immune response that was transferred to naive mice challenged with the EMT6 tumor; 9/10 or 90% of mice that received PDT therapy developed a protective anti-tumor immune response. However, administration of M158, a muCD40L specific antibody that neutralizes CD40L biological activity, to mice after PDT therapy prevented the development of a protective anti-tumor immune response in 60% of mice. The control rat IgG protein did not alter the development of PDT-induced anti-tumor immunity. Surgical removal of the tumor did not induce a protective immune response either.

These findings indicate that CD40L function is required for development of a protective anti-tumor immune response that occurs following PDT treatment. That CD40L is biologically important in the generation of PDT-induced protective anti-tumor immunity *in vivo,* is a significant and novel observation. Since PDT is a unique treatment, it can be used when surgery, chemotherapy and/or radiation have not eliminated the cancer. Combining PDT with administration of a CD40 binding protein should significantly enhance the *in vivo* anti-tumor immune response to a variety of tumors.

### EXAMPLE 2

This example demonstrates that the administration of a CD40 binding protein (soluble, trimeric CD40L referred to as CD40LT) to tumor-bearing subjects in conjunction with PDT *in vivo* enhances anti-tumor treatment. Female BALB/c mice are inoculated subcutaneously (SQ) with tumor cells derived from a weakly immunogenic tumor. The tumor cells are allowed to grow for a time sufficient to establish a tumor that cannot be totally eradicated with PDT; a portion of the mice are injected intraperitoneally (IP) with Photofrin® (a photosensitizer obtained from QLT, Vancouver, Canada) at a dose of 5.0 mg/kg. The remaining tumor-bearing mice are not given Photofrin® (negative control). The day following Photofrin® administration, half of the Photofrin-injected, tumor-bearing mice are treated with 135 J/cm² of red light having a wavelength of 630 nm. Tumors from the remaining Photofrin-injected, tumor-bearing mice, which were not exposed to the red light, are surgically removed. Within one to two days following light treatment or surgery, half of the mice in each treatment group receive, IP, 200 µg of rat IgG (Sigma), or CD40LT. Tumor incidence and tumor growth is monitored as needed. The table below presents a treatment matrix used to allocate an appropriate number of mice to each group.

**Table 2:**

| Evaluation of CD40LT/PDT CombinationTherapy | |
|---|---|
| Tumor Therapy | Antibody Treatment |
| None | Rat IgG |
| None | CD40LT |
| PDT | Rat IgG |
| PDT | CD40LT |
| Surgical removal | Rat IgG |
| Surgical removal | CD40LT |

## Claims

1. A kit of parts comprising a CD40 binding protein and a photosensitizer for separate, simultaneous or subsequent administration to a subject for the treatment of a tumour or a precancerous condition.

2. A kit of parts as claimed in claim 1, wherein the kit additionally comprises an active agent selected from the group consisting of:
a) FasL;
b) CD30L;
c) TRAIL; and
d) TNF alpha

3. A kit of parts as claimed in claim 1 or claim 2, wherein said CD40 binding protein is selected from the group consisting of:
a) an antibody to CD40;
b) CD40L;
c) soluble CD40L; and
d) an oligomeric soluble CD40L fusion protein comprising 1) a soluble CD40L or an antibody to CD40, and 2) a second protein.

4. A kit of parts as claimed in claim 3, wherein said antibody to CD40 is selected from the group consisting of monoclonal antibody HuCD40-M2 (ATCC HB11459) and antibodies having an antigen binding domain of antibody HuCD40M2.

5. A kit of parts as claimed in any one of claims 1 to 4, wherein said CD40 binding protein is selected from the group consisting of:
(a) a polypeptide comprising amino acids 1 through 260 of SEQ ID NO:1, 47 through 260 of SEQ ID NO:1, 113 through 260 of SEQ ID NO:1, or 120 through 260 of SEQ ID NO:1;
(b) a polypeptide comprising amino acids 1 through 261 of SEQ m NO:2, 47 through 261 of SEQ ID NO:2, 112 through 261 of SEQ ID NO:2, 113 through 261 of SEQ m NO:2, or 120 through 261 of SEQ ID NO:2;
(c) a polypeptide comprising a fragment of the polypeptides of SEQ ID NO:1, wherein the fragment binds CD40;
(d) a polypeptide comprising a fragment of the polypeptides of SEQ ID NO:2, wherein the fragment binds CD40;
(e) a polypeptide according to (b) or (c) wherein the cysteine at amino acid 194 of SEQ ID NO:2 is substituted with trytophan; and
(f) a polypeptide, encoded by the complement of a DNA that hybridizes to a DNA encoding any of the polypeptides of (a)-(e) under conditions of severe stringency (hybridization in 6 X SSC at 63°C overnight; washing in 3 X SSC at 55°C), wherein the encoded polypeptide binds CD40.

6. A kit of parts as claimed in claim 3, wherein said second protein is selected from the group consisting of an immunoglobulin Fc domain and an oligomerizing zipper domain.

7. A kit of parts as claimed in claim 6, wherein the oligomerizing zipper domain is selected from the group consisting of:
(a) a peptide having an amino acid sequence represented by SEQ ID NO: 3; and
(b) a variant of the peptide of (a), wherein the variant consists essentially of the peptide of (a) with one or more conservative amino acid substitutions, wherein the variant is capable of forming an oligomeric CD40L fusion protein.

8. A kit of parts as claimed in claim 1, wherein the CD40 binding protein comprises amino acids 113 through 261 of SEQ ID NO:2 and the peptide of SEQ ID No: 3.

9. A kit of parts as claimed in claim 3, wherein the oligomeric soluble CD40L comprises amino acids 113-261 of SEQ ID NO:2 and the peptide of SEQ ID NO:3, wherein the cysteine at amino acid 194 of SEQ ID NO:2 is substituted with tryptophan.

10. A kit of parts as claimed in any one of claims 1 to 9, wherein said tumor is selected from the group consisting of a B-lymphoma, a melanoma and a carcinoma.

11. The use of a CD40 binding protein in the preparation of a medicament for the photodynamic treatment of a subject suffering from a tumour or pre-cancerous condition.

12. A use as claimed in claim 11 wherein the medicament additionally comprises an active agent selected from the group consisting of:
e) FasL;
f) CD30L;
g) TRAIL; and
h) TNF alpha

13. A use as claimed in claim 11, wherein said CD40 binding protein is selected from the group consisting of:
e) an antibody to CD40;
f) CD40L;
g) soluble CD40L; and
h) an oligomeric soluble CD40L fusion protein comprising 1) a soluble CD40L or an antibody to CD40, and 2) a second protein.

14. A use as claimed in claim 13, wherein said antibody to CD40 is selected from the group consisting of monoclonal antibody HuCD40-M2 (ATCC HB 11459) and antibodies having an antigen binding domain of antibody HuCD40M2.

15. A use as claimed in any one of claims 11 to 14, wherein said CD40 binding protein is selected from the group consisting of:
(g) a polypeptide comprising amino acids 1 through 260 of SEQ ID NO:1, 47 through 260 of SEQ ID NO:1,113 through 260 of SEQ ID NO:1, or 120 through 260 of SEQ ID NO: 1;
(h) a polypeptide comprising amino acids 1 through 261 of SEQ ID NO:2, 47 through 261 of SEQ ID NO:2, 112 through 261 of SEQ ID NO:2, 113 through 261 of SEQ ID NO:2, or 120 through 261 of SEQ ID NO:2;
(i) a polypeptide comprising a fragment of the polypeptides of SEQ ID NO:1, wherein the fragment binds CD40;
(j) a polypeptide comprising a fragment of the polypeptides of SEQ ID NO:2, wherein the fragment binds CD40;
(k) a polypeptide according to (b) or (c) wherein the cysteine at amino acid 194 of SEQ ID NO:2 is substituted with trytophan; and
(l) a polypeptide, encoded by the complement of a DNA that hybridizes to a DNA encoding any of the polypeptides of (a)-(e) under conditions of severe stringency (hybridization in 6 X SSC at 63°C overnight; washing in 3 X SSC at 55°C), wherein the encoded polypeptide binds CD40.

16. A use as claimed in claim 13, wherein said second protein is selected from the group consisting of an immunoglobulin Fc domain and an oligomerizing zipper domain.

17. A use as claimed in claim 16, wherein the oligomerizing zipper domain is selected from the group consisting of:
(c) a peptide having an amino acid sequence represented by SEQ ID NO: 3; and
(d) a variant of the peptide of (a), wherein the variant consists essentially of the peptide of (a) with one or more conservative amino acid substitutions, wherein the variant is capable of forming an oligomeric CD40L fusion protein.

18. A use as claimed in claim 11 wherein the CD40 binding protein comprises amino acids 113 through 261 of SEQ ID NO:2 and the peptide of SEQ ID No: 3.

19. A use as claimed in claim 13, wherein the oligomeric soluble CD40L comprises amino acids 113-261 of SEQ ID NO:2 and the peptide of SEQ ID NO:3, wherein the cysteine at amino acid 194 of SEQ ID NO:2 is substituted with tryptophan.

20. A use as claimed in any one of claims 11 to 19, wherein said tumor is selected from the group consisting of a B-lymphoma, a melanoma and a carcinoma.

21. A kit of parts comprising a CD40 binding protein and a photosensitizer for separate, simultaneous or subsequent administration to a subject for inducing a memory CTL response in a tumor-bearing subject, wherein the memory CTL response is specific to the tumor.

22. A kit of parts as claimed in claim 21, wherein said CD40 binding protein is selected from the group consisting of:
(a) an antibody to CD40;
(b) CD40L;
(c) soluble CD40L; and
(d) an oligomeric soluble CD40L fusion protein comprising 1) a soluble CD40L or an antibody to CD40, and 2) a second protein.

23. A kit of parts as claimed in claim 22, wherein the CD40 binding protein is selected from the group consisting of:
(a) polypeptide comprising amino acids 1 through 260 of SEQ ID NO:1, amino acids 47 through 260 of SEQ ID NO:1, amino acids 113 through 260 of SEQ m NO:1, or amino acids 120 through 260 of SEQ ID NO:1;
(b) a polypeptide comprising amino acids 1 through 261 of SEQ ID NO:2, amino acids 47 through 261 of SEQ ID NO:2, amino acids 112 through 261 SEQ ID NO:2 of SEQ ID NO:2, amino acids 113 through 261 of SEQ ID NO:2, or amino acids 120 through 261 of SEQ ID NO:2;
(c) a polypeptide comprising a fragment of amino acids 47-260 of SEQ ID NO:1, wherein the fragment binds CD40;
(d) a polypeptide comprising a fragment of amino acids 47-261 of SEQ ID NO:2, wherein the fragment binds CD40;
(e) a polypeptide of (b) or (d), wherein the cysteine at amino acid 194 is substituted with tryptophan; and
(f) a polypeptide comprising an amino acid sequence that is encoded by the complement of a DNA that hybridizes to a DNA encoding any of the polypeptides of (a)-(f) under conditions of severe stringency (hybridization in 6 X SSC at 63°C overnight; washing in 3 X SSC at 55°C), wherein the encoded polypeptide binds soluble CD40.

24. A kit of parts as claimed in claim 22, wherein said second protein is selected from the group consisting of an immunoglobulin Fc domain and an oligomerizing zipper domain.

25. A kit of parts as claimed in claim 24, wherein the oligomerizing zipper domain is selected from the group consisting of:
(a) a polypeptide comprising the amino acids sequence of SEQ ID NO:3; and,
(b) a variant of the peptide of (a), wherein the variant consists essentially of the peptide of (a) with one or more conservative amino acid substitutions, wherein the variant is capable of forming an oligomeric CD40L fusion protein.

26. A kit of parts as claimed in claim 22, wherein the oligomeric soluble CD40L comprises amino acids 113-261 of SEQ ID NO:2 and the peptide of SEQ ID NO:3, wherein the cysteine at amino acid 194 of SEQ ID NO:2 is substituted with tryptophan.

27. A kit of parts as claimed in any one of claims 1 to 10, wherein the kit additionally comprises Flt3L.

28. A kit of parts as claimed in claim 1 or claim 27 further including Flt3L for treating hematopoietic stem or progenitor cells from the subject to obtain dendritic cells.

29. A kit of parts as claimed in any one of claim 1, claims 2 to 10, or claim 27 in combination with dendritic cells.

30. The use of a CD40 binding protein in the preparation of a medicament for inducing a memory CTL response in a tumour-bearing subject in conjunction with photodynamic therapy, wherein the memory CTL response is specific to the tumour.

31. A use as claimed in claim 30, wherein said CD40 binding protein is selected from the group consisting of:
(e) an antibody to CD40;
(f) CD40L;
(g) soluble CD40L; and
(h) an oligomeric soluble CD40L fusion protein comprising 1) a soluble CD40L or an antibody to CD40, and 2) a second protein.

32. A use as claimed in claim 31, wherein the CD40 binding protein is selected from the group consisting of:
(g) polypeptide comprising amino acids 1 through 260 of SEQ ID NO:1, amino acids 47 through 260 of SEQ ID NO:1, amino acids 113 through 260 of SEQ ID NO:1, or amino acids 120 through 260 of SEQ ID NO:1;
(h) a polypeptide comprising amino acids 1 through 261 of SEQ ID NO:2, amino acids 47 through 261 of SEQ ID NO:2, amino acids 112 through 261 SEQ ID NO:2 of SEQ ID NO:2, amino acids 113 through 261 of SEQ ID NO:2, or amino acids 120 through 261 of SEQ ID NO:2;
(i) a polypeptide comprising a fragment of amino acids 47-260 of SEQ ID NO:1, wherein the fragment binds CD40;
(j) a polypeptide comprising a fragment of amino acids 47-261 of SEQ ID NO:2, wherein the fragment binds CD40;
(k) a polypeptide of (b) or (d), wherein the cysteine at amino acid 194 is substituted with tryptophan; and
(l) a polypeptide comprising an amino acid sequence that is encoded by the complement of a DNA that hybridizes to a DNA encoding any of the polypeptides of (a)-(f) under conditions of severe stringency (hybridization in 6 X SSC at 63°C overnight; washing in 3 X SSC at 55°C), wherein the encoded polypeptide binds soluble CD40.

33. A use as claimed in claim 31, wherein said second protein is selected from the group consisting of an immunoglobulin Fc domain and an oligomerizing zipper domain.

34. A use as claimed in claim 33, wherein the oligomerizing zipper domain is selected from the group consisting of:
(c) a polypeptide comprising the amino acids sequence of SEQ ID NO:3; and,
(d) a variant of the peptide of (a), wherein the variant consists essentially of the peptide of (a) with one or more conservative amino acid substitutions, wherein the variant is capable of forming an oligomeric CD40L fusion protein.

35. A use as claimed in claim 31, wherein the oligomeric soluble CD40L comprises amino acids 113-261 of SEQ ID NO:2 and the peptide of SEQ ID NO:3, wherein the cysteine at amino acid 194 of SEQ ID NO:2 is substituted with tryptophan.

36. A use as claimed in any one of claims 11 to 20, wherein the kit or medicament additionally comprises FIt3L.

37. A use as claimed in claim 11 or claim 36 further including FIt3L for treating hematopoietic stem or progenitor cells from the subject to obtain dendritic cells.

## Patentansprüche

1. Satz von Teilen, der ein CD40-Bindeprotein und einen Fotosensibilisator zur getrennten, gleichzeitigen oder aufeinander folgenden Verabreichung an eine Person zur Behandlung eines Tumors oder eines präkanzerösen Zustandes aufweist.

2. Satz von Teilen nach Anspruch 1, wobei der Satz außerdem einen Wirkstoff enthält, der aus der Gruppe bestehend aus:
a) FasL,
b) CD30L,
c) TRAIL und
d) TNF-Alpha
ausgewählt ist.

3. Satz von Teilen nach Anspruch 1 oder Anspruch 2, wobei das genannte CD40-Bindeprotein aus der Gruppe bestehend aus:
a) einem Antikörper gegen CD40,
b) CD40L,
c) löslichem CD40L und
d) einem oligomeren löslichen CD40L-Fusionsprotein, das 1) ein lösliches CD40L oder einen Antikörper gegen CD40, und 2) ein zweites Protein umfasst,
ausgewählt ist.

4. Satz von Teilen nach Anspruch 3, wobei der genannte Antikörper gegen CD40 aus der Gruppe bestehend aus monoklonalem Antikörper HuCD40-M2 (ATCC HB11459) und Antikörpern, die eine Antigenbindungsdomäne von Antikörper HuCD40M2 besitzen, ausgewählt ist.

5. Satz von Teilen nach einem der Ansprüche 1 bis 4, wobei das CD40-Bindeprotein aus der Gruppe bestehend aus:
a) einem Polypeptid, das die Aminosäuren 1 bis 260 von SEQ ID NO:1, 47 bis 260 von SEQ ID NO:1, 113 bis 260 von SEQ ID NO:1 oder 120 bis 260 von SEQ ID NO:1 aufweist,
b) einem Polypeptid, das die Aminosäuren 1 bis 261 von SEQ ID NO:2, 47 bis 261 von SEQ ID NO:2, 112 bis 261 von SEQ ID NO:2, 113 bis 261 von SEQ ID NO:2 oder 120 bis 261 von SEQ ID NO:2 aufweist,
c) einem Polypeptid, das ein Fragment der Polypeptide von SEQ ID NO:1 aufweist, wobei das Fragment CD40 bindet,
d) einem Polypeptid, das ein Fragment der Polypeptide von SEQ ID NO:2 aufweist, wobei das Fragment CD40 bindet,
e) einem Polypeptid gemäß b) oder c), wobei das Cystein bei Aminosäure 194 von SEQ ID NO:2 durch Tryptophan ersetzt ist, und
f) einem Polypeptid, das durch das Komplement einer DNA codiert wird, die mit einer DNA hybridisiert, welche eines der Polypeptide von a)-c) unter strenger Stringenz (Hybridisierung über Nacht in 6 x SSC bei 63 °C; Waschung in 3 x SSC bei 55°C) codiert, wobei das codierte Polypeptid CD40 bindet,
ausgewählt ist.

6. Satz von Teilen nach Anspruch 3, wobei das genannte zweite Protein aus der Gruppe bestehend aus einer Immunglobulin Fc-Domäne und einer oligomerisierenden Zipper-Domäne ausgewählt ist.

7. Satz von Teilen nach Anspruch 6, wobei die oligomerisierende Zipper-Domäne aus der Gruppe bestehend aus
a) einem Peptid, das eine durch SEQ ID NO:3 repräsentierte Aminosäuresequenz besitzt, und
b) einer Variante des Peptids von a), wobei die Variante im wesentlichen aus dem Peptid von a) mit einer oder mehreren konservativen Aminosäuresubstitutionen besteht, wobei die Variante in der Lage ist, ein oligomeres CD40L-Fusionsprotein zu bilden.
ausgewählt ist.

8. Satz von Teilen nach Anspruch 1, wobei das CD40-Bindeprotein die Aminosäuren 113 bis 261 von SEQ ID NO:2 und das Peptid von SEQ ID NO:3 aufweist.

9. Satz von Teilen nach Anspruch 3, wobei das oligomere lösliche CD40L die Aminosäuren 113 bis 261 von SEQ ID NO:2 und das Peptid von SEQ ID NO:3 aufweist, wobei das Cystein bei Aminosäure 194 von SEQ ID NO:2 durch Tryptophan ersetzt ist.

10. Satz von Teilen nach einem der Ansprüche 1 bis 9, wobei der genannte Tumor aus der Gruppe bestehend aus einem B-Lymphom, einem Melanom und einem Karzinom ausgewählt ist.

11. Verwendung eines CD40-Bindeproteins bei der Herstellung eines Medikamentes zur fotodynamischen Behandlung einer Person, die an einem Tumor oder einem präkanzerösen Zustand leidet.

12. Verwendung nach Anspruch 11, wobei das Medikament weiters einen Wirkstoff aufweist, der aus der Gruppe bestehend aus:
a) FasL,
b) CD30L,
c) TRAIL und
d) TNF-Alpha
ausgewählt ist.

13. Verwendung nach Anspruch 11, wobei das genannte CD40-Bindeprotein aus der Gruppe bestehend aus
a) einem Antikörper gegen CD40,
b) CD40L,
c) löslichem CD40L und
d) einem oligomeren löslichen CD40L-Fusionsprotein, das 1) ein lösliches CD40L oder einen Antikörper gegen CD40, und 2) ein zweites Protein aufweist,
ausgewählt ist.

14. Verwendung nach Anspruch 13, wobei der genannte Antikörper gegen CD40 aus der Gruppe bestehend aus monoklonalem Antikörper HuCD40-M2 (ATCC HB11459) und Antikörpern, die eine Antigenbindungsdomäne von Antikörper HuCD40M2 besitzen, ausgewählt ist.

15. Verwendung nach einem der Ansprüche 11 bis 14, wobei das genannte CD40-Bindeprotein aus der Gruppe bestehend aus:
g) einem Polypeptid, das die Aminosäuren 1 bis 260 von SEQ ID NO:1, 47 bis 260 von SEQ ID NO:1, 113 bis 260 von SEQ ID NO:1 oder 120 bis 260 von SEQ ID NO:1 aufweist,
h) einem Polypeptid, das die Aminosäuren 1 bis 261 von SEQ ID NO:2, 47 bis 261 von SEQ ID NO:2, 112 bis 261 von SEQ ID NO:2, 113 bis 261 von SEQ ID NO:2 oder 120 bis 261 von SEQ ID NO:2 aufweist,
i) einem Polypeptid, das ein Fragment der Polypeptide von SEQ ID NO:1 aufweist, wobei das Fragment CD40 bindet,
j) einem Polypeptid, das ein Fragment der Polypeptide von SEQ ID NO:2 aufweist, wobei das Fragment CD40 bindet,
k) einem Polypeptid gemäß b) oder c), wobei das Cystein bei Aminosäure 194 von SEQ ID NO:2 durch Tryptophan ersetzt wird, und
l) einem Polypeptid, das durch das Komplement einer DNA codiert wird, die mit einer DNA hybridisiert, welche eines der Polypeptide von a)-c) unter strenger Stringenz (Hybridisierung über Nacht in 6 x SSC bei 63 °C; Waschung in 3 x SSC bei 55 °C) codiert, wobei das codierte Polypeptid CD40 bindet,
ausgewählt ist.

16. Verwendung nach Anspruch 13, wobei das genannte zweite Protein aus der Gruppe bestehend aus einer Immunglobulin Fc-Domäne und einer oligomerisierenden Zipper-Domäne ausgewählt ist.

17. Verwendung nach Anspruch 16, wobei die oligomerisierende Zipper-Domäne aus der Gruppe bestehend aus:
c) einem Peptid mit einer Aminosäuresequenz, die durch SEQ ID NO:3 repräsentiert wird, und
d) einer Variante des Peptids von a), wobei die Variante im wesentlichen aus dem Peptid von a) mit einer oder mehreren konservativen Aminosäuresubstitutionen besteht, wobei die Variante in der Lage ist, ein oligomeres CD40L-Fusionsprotein zu bilden,
ausgewählt ist.

18. Verwendung nach Anspruch 11, wobei das CD40-Bindeprotein die Aminosäuren 113 bis 261 von SEQ ID NO:2 und das Peptid von SEQ ID NO:3 aufweist.

19. Verwendung nach Anspruch 13, wobei das oligomere lösliche CD40L die Aminosäuren 113-261 von SEQ ID NO:2 und das Peptid von SEQ ID NO:3 aufweist, wobei das Cystein bei Aminosäure 194 von SEQ ID NO:2 durch Tryptophan ersetzt ist.

20. Verwendung nach einem der Ansprüche 11 bis 19, wobei der genannte Tumor aus der Gruppe bestehend aus einem B-Lymphom, einem Melanom und einem Karzinom ausgewählt ist.

21. Satz von Teilen, das ein CD40-Bindeprotein und einen Fotosensibilisator zur getrennten, gleichzeitigen oder aufeinander folgenden Verabreichung an eine Person zur Induzierung einer CTL-Immunreaktion in einer tumortragenden Person aufweist, wobei die CTL-Immunreaktion spezifisch für den Tumor ist.

22. Satz von Teilen nach Anspruch 21, wobei das genannte CD40-Bindeprotein aus der Gruppe bestehend aus:
a) einem Antikörper gegen CD40,
b) CD40L,
c) löslichem CD40L und
d) einem oligomeren löslichen CD40L-Fusionsprotein, das 1) ein lösliches CD40L oder einen Antikörper gegen CD40, und 2) ein zweites Protein aufweist,
ausgewählt ist.

23. Satz von Teilen nach Anspruch 22, wobei das CD40-Bindeprotein aus der Gruppe bestehend aus:
a) einem Polypeptid, das die Aminosäuren 1 bis 260 von SEQ ID NO:1, die Aminosäuren 47 bis 260 von SEQ ID NO:1, die Aminosäuren 113 bis 260 von SEQ ID NO:1 oder die Aminosäuren 120 bis 260 von SEQ ID NO:1 aufweist,
b) einem Polypeptid, das die Aminosäuren 1 bis 261 von SEQ ID NO:2, die Aminosäuren 47 bis 261 von SEQ ID NO:2, die Aminosäuren 112 bis 261 von SEQ ID NO:2, die Aminosäuren 113 bis 261 von SEQ ID NO:2 oder die Aminosäuren 120 bis 261 von SEQ ID NO:2 aufweist,
c) einem Polypeptid, das ein Fragment der Aminosäuren 47-260 von SEQ ID NO:1 aufweist, wobei das Fragment CD40 bindet,
d) einem Polypeptid, das ein Fragment der Aminosäuren 47-261 von SEQ ID NO:2 aufweist, wobei das Fragment CD40 bindet,
e) einem Polypeptid gemäß b) oder d), wobei das Cystein bei Aminosäure 194 durch Tryptophan ersetzt ist, und
f) einem Polypeptid, das eine Aminosäuresequenz aufweist, die durch das Komplement einer DNA codiert wird, welche mit einer DNA hybridisiert, die eines der Polypeptide von a)-f) unter strenger Stringenz (Hybridisierung über Nacht in 6 x SSC bei 63 °C; Waschung in 3 x SSC bei 55 °C) codiert, wobei das codierte Polypeptid lösliches CD40 bindet,
ausgewählt ist.

24. Satz von Teilen nach Anspruch 22, wobei das genannte zweite Protein aus der Gruppe bestehend aus einer Immunglobulin Fc-Domäne und einer oligomerisierenden Zipper-Domäne ausgewählt ist.

25. Satz von Teilen nach Anspruch 24, wobei die oligomerisierende Zipper-Domäne aus der Gruppe bestehend aus:
a) einem Polypeptid, das die Aminosäuresequenz von SEQ ID NO:3 aufweist, und
b) einer Variante des Peptids von a), wobei die Variante im wesentlichen aus dem Peptid von a) mit einer oder mehreren konservativen Aminosäuresubstitutionen besteht, wobei die Variante in der Lage ist, ein oligomeres CD40L-Fusionsprotein zu bilden,
ausgewählt ist.

26. Satz von Teilen nach Anspruch 22, wobei das oligomere lösliche CD40L die Aminosäuren 113-261 von SEQ ID NO:2 und das Peptid von SEQ ID NO:3 aufweist, wobei das Cystein bei Aminosäure 194 von SEQ ID NO:2 durch Tryptophan ersetzt ist.

27. Satz von Teilen nach einem der Ansprüche 1 bis 10, wobei der Satz außerdem Flt3L umfasst.

28. Satz von Teilen nach Anspruch 1 oder Anspruch 27, das weiters Flt3L zur Behandlung von hämatopoietischen Stamm- oder Vorläuferzellen enthält, die der Person entnommen wurden, um dendritische Zellen zu gewinnen.

29. Satz von Teilen nach Anspruch 1, Ansprüchen 2 bis 10 oder Anspruch 27 in Kombination mit dendritischen Zellen.

30. Verwendung eines CD40-Bindeproteins bei der Herstellung eines Medikamentes zur Induzierung einer CTL-Immunreaktion in einer tumortragenden Person in Verbindung mit fotodynamischer Therapie, wobei die CTL-Immunreaktion spezifisch für den Tumor ist.

31. Verwendung nach Anspruch 30, wobei das genannte CD40-Bindeprotein aus der Gruppe bestehend aus:
e) einem Antikörper gegen CD40,
f) CD40L,
g) löslichem CD40L und
h) einem oligomeren löslichen CD40L-Fusionsprotein, das 1) ein lösliches CD40L oder einen Antikörper gegen CD40, und 2) ein zweites Protein enthält,
ausgewählt ist.

32. Verwendung nach Anspruch 31, wobei das CD40-Bindeprotein aus der Gruppe bestehend aus:
g) einem Polypeptid, das die Aminosäuren 1 bis 260 von SEQ ID NO:1, die Aminosäuren 47 bis 260 von SEQ ID NO:1, die Aminosäuren 113 bis 260 von SEQ ID NO:1 oder die Aminosäuren 120 bis 260 von SEQ ID NO:1 aufweist,
h) einem Polypeptid, das die Aminosäuren 1 bis 261 von SEQ ID NO:2, die Aminosäuren 47 bis 261 von SEQ ID NO:2, die Aminosäuren 112 bis 261 von SEQ ID NO:2, die Aminosäuren 113 bis 261 von SEQ ID NO:2 oder die Aminosäuren 120 bis 261 von SEQ ID NO:2 aufweist,
i) einem Polypeptid, das ein Fragment der Aminosäuren 47-260 von SEQ ID NO:1 aufweist, wobei das Fragment CD40 bindet,
j) einem Polypeptid, das ein Fragment der Aminosäuren 47-261 von SEQ ID NO:2 aufweist, wobei das Fragment CD40 bindet,
k) einem Polypeptid gemäß b) oder d), wobei das Cystein bei Aminosäure 194 durch Tryptophan ersetzt ist, und
l) einem Polypeptid, das eine Aminosäuresequenz aufweist, die durch das Komplement einer DNA codiert wird, welche mit einer DNA hybridisiert, die eines der Polypeptide von a)-f) unter strenger Stringenz (Hybridisierung über Nacht in 6 x SSC bei 63 °C; Waschung in 3 x SSC bei 55 °C) codiert, wobei das codierte Polypeptid lösliches CD40 bindet,
ausgewählt ist.

33. Verwendung nach Anspruch 31, wobei das genannte zweite Protein aus der Gruppe bestehend aus einer Immunglobulin Fc-Domäne und einer oligomerisierenden Zipper-Domäne ausgewählt ist.

34. Verwendung nach Anspruch 33, wobei die oligomerisierende Zipper-Domäne aus der Gruppe bestehend aus:
c) einem Polypeptid, das die Aminosäuresequenz von SEQ ID NO:3 aufweist, und
d) einer Variante des Peptids von a), wobei die Variante im wesentlichen aus dem Peptid von a) mit einer oder mehreren konservativen Aminosäuresubstitutionen besteht, wobei die Variante in der Lage ist, ein oligomeres CD40L-Fusionsprotein zu bilden,
ausgewählt ist.

35. Verwendung nach Anspruch 31, wobei das oligomere lösliche CD40L die Aminosäuren 113-261 von SEQ ID NO:2 und das Peptid von SEQ ID NO:3 aufweist, wobei das Cystein bei Aminosäure 194 von SEQ ID NO:2 durch Tryptophan ersetzt ist.

36. Verwendung nach einem der Ansprüche 11 bis 20, wobei der Satz oder das Medikament weiters Flt3L enthält.

37. Verwendung nach Anspruch 11 oder Anspruch 36, die weiters Flt3L zur Behandlung von hämatopoietischen Stamm- oder Vorläuferzellen aus einer Person enthält, um dendritische Zellen zu gewinnen.

## Revendications

1. Kit d'éléments comprenant une protéine de liaison à CD40 et un photosensibilisateur destiné à l'administration séparée, simultanée ou subséquente à un sujet en vue du traitement d'une tumeur ou d'un état précancéreux.

2. Kit d'éléments selon la revendication 1, **caractérisé en ce que** le kit comprend en outre un principe actif choisi parmi le groupe constitué de :
a) FasL;
b) CD30L;
c) TRAIL; et
d) TNF alpha.

3. Kit d'éléments selon la revendication 1 ou la revendication 2, **caractérisé en ce que** ladite protéine de liaison à CD40 est choisie parmi le groupe constitué de :
a) un anticorps de CD40;
b) CD40L;
c) CD40L soluble; et
d) une protéine de fusion de CD40L soluble oligomère comprenant 1) une CD40L soluble ou un anticorps de CD40, et 2) une deuxième protéine.

4. Kit d'éléments selon la revendication 3, **caractérisé en ce que** ledit anticorps de CD40 est choisi parmi le groupe constitué de l'anticorps monoclonal HuCD40-M2 (ATCC HB11459) et d'anticorps ayant un domaine de liaison de l'anticorps HuCD40M2 à un antigène.

5. Kit d'éléments selon l'une quelconque des revendications 1 à 4, **caractérisé en ce que** ladite protéine de liaison à CD40 est choisie parmi le groupe constitué de :
(a) un polypeptide comprenant les acides aminés 1 à 260 de la SEQ ID NO:1, 47 à 260 de la SEQ ID NO:1, 113 à 260 de la SEQ ID NO:1 ou 120 à 260 de la SEQ ID NO:1;
(b) un polypeptide comprenant les acides aminés 1 à 261 de la SEQ ID NO:2, 47 à 261 de la SEQ ID NO:2, 112 à 261 de la SEQ ID NO:2, 113 à 261 de la SEQ ID NO:2 ou 120 à 261 de la SEQ ID NO:2;
(c) un polypeptide comprenant un fragment des polypeptides de la SEQ ID NO:1, le fragment se liant à CD40;
(d) un polypeptide comprenant un fragment des polypeptides de la SEQ ID NO:2, le fragment se liant à CD40;
(e) un polypeptide selon (b) ou (c), la cystéine à l'endroit de l'acide aminé 194 de la SEQ ID NO:2 étant substituée par le tryptophane; et
(f) un polypeptide codé par le complément d'un ADN qui s'hybride avec un ADN codant pour l'un quelconque des polypeptides de (a)-(e) dans des conditions de stringence stricte (hybridation dans 6 X SSC à 63°C pendant une nuit; lavage dans 3 X SSC à 55°C), le polypeptide codé se liant à la CD40.

6. Kit d'éléments selon la revendication 3, **caractérisé en ce que** ladite deuxième protéine est choisie parmi le groupe constitué d'un domaine Fc d'immunoglobuline et d'un domaine glissière d'oligomérisation.

7. Kit d'éléments selon la revendication 6, **caractérisé en ce que** le domaine glissière d'oligomérisation est choisi parmi le groupe constitué de :
(a) un peptide ayant une séquence d'acides aminés représentée par la SEQ ID NO:3; et
(b) un variant du peptide de (a), le variant étant essentiellement constitué du peptide de (a) avec une ou plusieurs substitutions conservatrices d'acides aminés, le variant étant capable de former une protéine de fusion de CD40L oligomère.

8. Kit d'éléments selon la revendication 1, **caractérisé en ce que** la protéine de liaison à CD40 comprend les acides aminés 113 à 261 de la SEQ ID NO:2 et le peptide de la SEQ ID NO:3.

9. Kit d'éléments selon la revendication 3, **caractérisé en ce que** la CD40L soluble oligomère comprend les acides aminés 113-261 de la SEQ ID NO:2 et le peptide de la SEQ ID NO:3, la cystéine à l'endroit de l'acide aminé 194 de la SEQ ID NO:2 étant substituée par le tryptophane.

10. Kit d'éléments selon l'une quelconque des revendications 1 à 9 **caractérisé en ce que** ladite tumeur est choisie parmi le groupe constitué d'un lymphome B, d'un mélanome et d'un carcinome.

11. Utilisation d'une protéine de liaison à CD40 dans la préparation d'un médicament pour le traitement photodynamique d'un sujet souffrant d'une tumeur ou d'un état précancéreux.

12. Utilisation selon la revendication 11, **caractérisée en ce que** le médicament comprend en outre un principe actif choisi parmi le groupe constitué de :
e) FasL;
f) CD30L;
g) TRAIL; et
h) TNF alpha.

13. Utilisation selon la revendication 11, **caractérisée en ce que** ladite protéine de liaison à CD40 est choisie parmi le groupe constitué de :
e) un anticorps de CD40;
f) CD40L;
g) CD40L soluble; et
h) une protéine de fusion de CD40L soluble oligomère comprenant 1) une CD40L soluble ou un anticorps de CD40 et 2) une deuxième protéine.

14. Utilisation selon la revendication 13, **caractérisée en ce que** ledit anticorps de CD40 est choisi parmi le groupe constitué de l'anticorps monoclonal HuCD40-M2 (ATCC HB11459) et d'anticorps ayant un domaine de liaison de l'anticorps HuCD40M2 à un antigène.

15. Utilisation selon l'une quelconque des revendications 11 à 14, **caractérisée en ce que** ladite protéine de liaison à CD40 est choisie parmi le groupe constitué de :
(g) un polypeptide comprenant les acides aminés 1 à 260 de la SEQ ID NO:1, 47 à 260 de la SEQ ID NO:1, 113 à 260 de la SEQ ID NO:1 ou 120 à 260 de la SEQ ID NO:1;
(h) un polypeptide comprenant les acides aminés 1 à 261 de la SEQ ID NO:2, 47 à 261 de la SEQ ID NO:2, 112 à 261 de la SEQ ID NO:2, 113 à 261 de la SEQ ID NO:2 ou 120 à 261 de la SEQ ID NO:2;
(i) un polypeptide comprenant un fragment des polypeptides de la SEQ ID NO:1, le fragment se liant à CD40;
(j) un polypeptide comprenant un fragment des polypeptides de la SEQ ID NO:2, le fragment se liant à CD40;
(k) un polypeptide selon (b) ou (c), la cystéine à l'endroit de l'acide aminé 194 de la SEQ ID NO:2 étant substituée par le tryptophane; et
(l) un polypeptide, codé par le complément d'un ADN qui s'hybride avec un ADN codant pour l'un quelconque des polypeptides de (a)-(e) dans des conditions de stringence stricte (hybridation dans 6 X SSC à 63°C pendant une nuit ; lavage dans 3 X SSC à 55°C), le polypeptide codé se liant à la CD40.

16. Utilisation selon la revendication 13, **caractérisée en ce que** ladite deuxième protéine est choisie parmi le groupe constitué d'un domaine Fc d'immunoglobuline et d'un domaine glissière d'oligomérisation.

17. Utilisation selon la revendication 16, **caractérisée en ce que** le domaine glissière d'oligomérisation est choisi parmi le groupe constitué de :
(c) un peptide ayant une séquence d'acides aminés représentée par la SEQ ID NO:3; et
(d) un variant du peptide de (a), le variant étant essentiellement constitué du peptide de (a) avec une ou plusieurs substitutions conservatrices d'acides aminés, le variant étant capable de former une protéine de fusion de CD40L oligomère.

18. Utilisation selon la revendication 11, **caractérisée en ce que** la protéine de liaison à CD40 comprend les acides aminées 113 à 261 de la SEQ ID NO:2 et le peptide de la SEQ ID NO:3.

19. Utilisation selon la revendication 13, **caractérisée en ce que** la CD40L soluble oligomère comprend les acides aminés 113-261 de la SEQ ID NO:2 et le peptide de la SEQ ID NO:3, la cystéine à l'endroit de l'acide aminé 194 de la SEQ ID NO:2 étant substituée par le tryptophane.

20. Utilisation selon l'une quelconque des revendications 11 à 19, **caractérisée en ce que** ladite tumeur est choisie parmi le groupe constitué d'un lymphome B, d'un mélanome et d'un carcinome.

21. Kit d'éléments comprenant une protéine de liaison à CD40 et un photosensibilisateur destiné à l'administration séparée, simultanée ou subséquente à un sujet en vue d'induire une réponse CTL mémoire chez un sujet porteur d'une tumeur, la réponse CTL mémoire étant spécifique à la tumeur.

22. Kit d'éléments selon la revendication 21, **caractérisé en ce que** ladite protéine de liaison à CD40 est choisie parmi le groupe constitué de :
(a) un anticorps de CD40;
(b) CD40L;
(c) CD40L soluble; et
(d) une protéine de fusion de CD40L soluble oligomère comprenant 1) une CD40L soluble ou un anticorps de CD40 et 2) une deuxième protéine.

23. Kit d'éléments selon la revendication 22, **caractérisé en ce que** la protéine de liaison à CD40 est choisie parmi le groupe constitué de
(a) un polypeptide comprenant les acides aminés 1 à 260 de la SEQ ID NO:1, les acides aminés 47 à 260 de la SEQ ID NO:1, les acides aminés 113 à 260 de la SEQ ID NO:1 ou les acides aminés 120 à 260 de la SEQ ID NO:1;
(b) un polypeptide comprenant les acides aminés 1 à 261 de la SEQ ID NO:2, les acides aminés 47 à 261 de la SEQ ID NO:2, les acides aminés 112 à 261 de la SEQ ID NO:2, les acides aminés 113 à 261 de la SEQ ID NO:2 ou les acides aminés 120 à 261 de la SEQ ID NO:2;
(c) un polypeptide comprenant un fragment des acides aminés 47-260, de la SEQ ID NO:1, le fragment se liant à CD40;
(d) un polypeptide comprenant un fragment des acides aminés 47-261 de la SEQ ID NO:2, le fragment se liant à CD40;
(e) un polypeptide selon (b) ou (d), la cystéine à l'endroit de l'acide aminé 194 étant substituée par le tryptophane; et
(f) un polypeptide comprenant une séquence d'acides aminés qui est codée par le complément d'un ADN qui s'hybride avec un ADN codant pour l'un quelconque des polypeptides de (a)-(f) dans des conditions de stringence stricte (hybridation dans 6 X SSC à 63°C pendant une nuit; lavage dans 3 X SSC à 55°C), le polypeptide codé se liant à la CD40 soluble.

24. Kit d'éléments selon la revendication 22, **caractérisé en ce que** ladite deuxième protéine est choisie parmi le groupe constitué d'un domaine Fc d'immunoglobuline et d'un domaine glissière d'oligomérisation.

25. Kit d'éléments selon la revendication 24, **caractérisé en ce que** le domaine glissière d'oligomérisation est choisi parmi le groupe constitué de :
(a) un peptide comprenant la séquence d'acides aminés de la SEQ ID NO:3; et
(b) un variant du peptide de (a), le variant étant essentiellement constitué du peptide de (a) avec une ou plusieurs substitutions conservatrices d'acides aminés, le variant étant capable de former une protéine de fusion de CD40L oligomère.

26. Kit d'éléments selon la revendication 22, **caractérisé en ce que** la CD40L soluble oligomère comprend les acides aminés 113-261 de 1a SEQ ID NO:2 et le peptide de la SEQ ID NO:3, la cystéine à l'endroit de l'acide aminé 194 de la SEQ ID NO:2 étant substituée par le tryptophane.

27. Kit d'éléments selon l'une quelconque des revendications 1 à 10, **caractérisé en ce que** le kit comprend en outre le Flt3L.

28. Kit d'éléments selon la revendication 1 ou la revendication 27 comprenant en outre le Flt3L pour le traitement de cellules souches hématopoïétiques ou de cellules souches d'un sujet en vue d'obtenir des cellules dendritiques.

29. Kit d'éléments selon l'une quelconque des revendications 1, 2 à 10 ou 27 en combinaison avec des cellules dendritiques.

30. Utilisation d'une protéine de liaison à CD40 dans la préparation d'un médicament destiné à induire une réponse CTL mémoire chez un sujet porteur d'une tumeur conjointement avec une thérapie photodynamique, la réponse CTL mémoire étant spécifique à la tumeur.

31. Utilisation selon la revendication 30, **caractérisée en ce que** ladite protéine de liaison à CD40 est choisie parmi le groupe constitué de
(e) un anticorps de CD40;
(f) CD40L;
(g) CD40L soluble; et
(h) une protéine de fusion de CD40L soluble oligomère comprenant 1) une CD40L soluble ou un anticorps de CD40 et 2) une deuxième protéine.

32. Utilisation selon la revendication 31, **caractérisée en ce que** la protéine de liaison à CD40 est choisie parmi le groupe constitué de :
(g) un polypeptide comprenant les acides aminés 1 à 260 de la SEQ ID NO:1, les acides aminés 47 à 260 de la SEQ ID NO:1, les acides aminés 113 à 260 de la SEQ ID NO:1 ou les acides aminés 120 à 260 de la SEQ ID NO:1;
(h) un polypeptide comprenant les acides aminés 1 à 261 de la SEQ ID NO:2, les acides aminés 47 à 261 de la SEQ ID NO:2, les acides aminés 112 à 261 de la SEQ ID NO:2, les acides aminés 113 à 261 de la SEQ ID NO:2 ou les acides aminés 120 à 261 de la SEQ ID NO:2;
(i) un polypeptide comprenant un fragment des acides aminés 47-260 de la SEQ ID NO:1, le fragment se liant à CD40;
(j) un polypeptide comprenant un fragment des acides aminés 47-261 de la SEQ ID NO:2, le fragment se liant à CD40;
(k) un polypeptide selon (b) ou (d), la cystéine à l'endroit de l'acide aminé 194 étant substituée par le tryptophane; et
(l) un polypeptide comprenant une séquence d'acides aminés qui est codée par le complément d'un ADN qui s'hybride avec un ADN codant pour l'un quelconque des polypeptides de (a)-(f) dans des conditions de stringence stricte (hybridation dans 6 X SSC à 63°C pendant une nuit; lavage dans 3 X SSC à 55°C), le polypeptide codé se liant à une CD40 soluble.

33. Utilisation selon la revendication 31, **caractérisée en ce que** ladite deuxième protéine est choisie parmi le groupe constitué d'un domaine Fc d'immunoglobuline et d'un domaine glissière d'oligomérisation.

34. Utilisation selon la revendication 33, **caractérisée en ce que** le domaine glissière d'oligomérisation est choisi parmi le groupe constitué de :
(a) un polypeptide comprenant la séquence d'acides aminés de la SEQ ID NO:3; et
(b) un variant du peptide de (a), le variant étant essentiellement constitué du peptide de (a) avec une ou plusieurs substitutions conservatrices d'acides aminés, le variant étant capable de former une protéine de fusion de CD40L oligomère.

35. Utilisation selon la revendication 31, **caractérisée en ce que** la CD40L soluble oligomère comprend les acides aminés 113-261 de la SEQ ID NO:2 et le peptide de la SEQ ID NO:3, la cystéine à l'endroit de l'acide aminé 194 de la SEQ ID NO:2 étant substituée par le tryptophane.

36. Utilisation selon l'une quelconque des revendications 11 à 20, **caractérisée en ce que** le kit ou le médicament comprend en outre le Flt3L.

37. Utilisation selon la revendication 11 ou la revendication 36 comprenant en outre le Flt3L pour traiter des cellules souches hématopoïétiques ou des cellules souches d'un sujet en vue d'obtenir des cellules dendritiques.
